(19) **Europäisches Patentamt / European Patent Office / Office européen des brevets**

(11) **EP 2 005 188 B1**

(12) **EUROPEAN PATENT SPECIFICATION**

(45) Date of publication and mention
of the grant of the patent:
**16.06.2010 Bulletin 2010/24**

(51) Int Cl.:
*G01N 33/58* (2006.01)     *G01N 33/543* (2006.01)

(21) Application number: **07734105.5**

(22) Date of filing: **27.03.2007**

(86) International application number:
**PCT/IB2007/000779**

(87) International publication number:
**WO 2007/110756 (04.10.2007 Gazette 2007/40)**

(54) **ASSAY DEVICE AND METHOD**

TESTVORRICHTUNG UND VERFAHREN

DISPOSITIF ET PROCEDE DE DOSAGE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.03.2006 US 786363 P**

(43) Date of publication of application:
**24.12.2008 Bulletin 2008/52**

(73) Proprietor: **Inverness Medical Switzerland GmbH 6300 Zug (CH)**

(72) Inventors:
• **TAYLOR, David, William Alloa, Clackmannanshire FK10 2DT (GB)**
• **GILL, Andrew Alloa, Clackmannanshire FK10 2EF (GB)**

(74) Representative: **HOFFMANN EITLE Patent- und Rechtsanwälte Arabellastrasse 4 81925 München (DE)**

(56) References cited:
**WO-A-94/19690          WO-A1-2006/018811 US-A1- 2004 058 458**

## Description

## TECHNICAL FIELD

This invention relates to an assay device and method.

## BACKGROUND

A system for measuring a biological sample can use a replaceable cartridge or test strip and a reader. The cartridge accepts a sample and includes one or more reagents for producing a detectable change in the test sample. The detectable change can be related to the amount of an analyte in the sample. The cartridge reader can measure the detectable change and communicate a result to the user. The cartridge reader can calculate the amount of analyte in the sample (e.g. as a concentration of analyte in a liquid sample).

The system can be used by users who need to frequently measure an analyte. In particular, the system can be useful for patients with a chronic condition that requires monitoring. In order to encourage patient compliance with a monitoring regimen, it can be desirable for the system to require a small volume of sample and for the replaceable cartridges to be inexpensive.

US 2004/0058458 discloses modulated (e.g. magnetically modulated) chemical sensors. In particular, US 2004/0058458 discloses particles comprising fluorescent indicator dyes and methods of using such particles.

## SUMMARY

The present invention relates to assays.

In one aspect, an assay method includes forming a mixture by combining magnetically susceptible labels, optical (e.g., fluorescent) labels, and sample material including at least one analyte. The magnetic labels and optical labels are configured to form tertiary complexes with the analyte. The mixture is subjected to a magnetic field sufficient to move the tertiary complexes at a velocity different from (e.g., at a higher velocity than) optical labels not in a tertiary complex (e.g., free optical labels). First time-varying optical signals are received from optical labels in tertiary complexes and second time-varying signals are received from free optical labels. Because the tertiary complexes and the free optical labels move at different velocities, the frequency content of the first optical signals is different from the frequency content of the second optical signals. The presence of the analyte is determined based at least in part on the first optical signals.

In another aspect, an assay method includes forming a mixture by combining magnetically susceptible labels, optical (e.g., fluorescent) labels, and sample material including at least one analyte. The analyte and optical labels compete with one another to form binary complexes with the magnetic labels. The mixture is subjected to a magnetic field sufficient to move the binary complexes at a velocity different from (e.g., at a higher velocity than) optical labels not in a binary complex (e.g., free optical labels). First time-varying optical signals are received from optical labels in binary complexes and second time-varying signals are received from free optical labels. Because the binary complexes and the free optical labels move at different velocities, the frequency content of the first optical signals is different from the frequency content of the second optical signals. The presence of the analyte is determined based at least in part on the first optical signals.

In some embodiments, the first and second optical signals may be received by a detector that outputs an electronic signal indicative of the first and second optical signals. In some embodiments, the electronic signal is filtered electronically (e.g., through an electronic bandpass filter) to select (e.g., amplify) portions of the electronic signal that correspond to the first optical signal. The presence of the analyte is determined based at least in part on the selected portions of the electronic signal. In some embodiments, the electronic signal is autocorrelated. The presence of the analyte is determined based at least in part on portions of the autocorrelated signal that correspond to the first optical signal.

In some embodiments, the optical labels are fluorescent optical labels and the method includes illuminating at least an illuminated portion of the mixture with light sufficient to excite fluorescence from the fluorescent labels. The first optical signals include fluorescence emitted from fluorescent labels in tertiary complexes (or binary complexes) and the second optical signals include fluorescence emitted from free fluorescent labels.

In some embodiments, the illuminated portion of the mixture is illuminated with light have a spatially varying intensity. The intensity of fluorescence emitted by an optical label depends on its location within the illuminated portion of the mixture. Typically, the illuminating light intensity within the illuminated portion is configured to vary at an angle with respect to a direction of tertiary complex movement induced by the magnetic field. The fluorescence intensities of the first optical signals vary temporally as the tertiary complexes (or binary complexes) and free optical labels move through the illuminated portion. The frequency content of the optical signals depends on the velocity of the optical labels within the illuminated portion and the spatial variation of the illuminating light intensity. The first and second optical signals have different frequency contents because the tertiary complexes (or binary complexes) and free optical labels move at different velocities within the illuminated portion of the mixture.

In some embodiments, the illuminated portion of the mixture is illuminated with light that may have a generally constant (e.g., continuous) spatial variation within the illuminated portion. The first and second optical signals are received by a detector after passing along an optical path that has a spatially varying transmittance. While, the intensity of fluorescence emitted by an optical label is generally independent of its location within the illuminated portion of the mixture; the intensity of fluorescence received from an optical label depends on its location with respect to the spatially varying transmittance of the optical path. The frequency content of the optical signals depends on the velocity of the optical labels within the illuminated portion and the spatial variation of the optical path transmittance. The first and second optical signals have different frequency contents because the tertiary complexes (or binary complexes) and free optical labels move at different velocities with respect to the spatially varying optical path transmittance.

An assay system can include a replaceable assay device and an assay device reader. The assay device can take the form of a cartridge or test strip. The system can provide high sensitivity, low volume detection of an analyte in a sample. The assay device can be simple to manufacture.

In many assay devices, a compound or substance to be detected (i.e., the analyte) is bound by a labeled reagent. The label is creates a detectable change, which can be, for example, fluorescence or color. In order to reliably determine the amount of analyte in a sample, it can be important to distinguish the fraction of labeled reagent that is bound to analyte from the fraction of labeled reagent that remains unbound. The ratio of bound to unbound reagent can be related to the concentration of analyte in the sample.

The assay device can be supplied with a reagent linked to a magnetic particle that binds to the analyte at the same time as the labeled reagent. This allows the bound and free label to be distinguished by virtue of their behavior in a magnetic field. When both reagents are bound to the analyte via different epitopes, the resultant complex (i.e., bound label) can be moved in a magnetic field. If, however, the second reagent is not bound to the first reagent via the analyte (i.e., free label), the second reagent will not move in a magnetic field. Thus it is possible to distinguish the fraction of second reagent bound to the first reagent via the analyte from the unbound fraction of second reagent, and then to calculated the analyte concentration. The label can be detected by optical methods, such as fluorescence. The system can be simple for patients to use in the home.

In one embodiment, a device for determining a sample compound includes a detection zone configured to accommodate sample material. The detection zone includes a magnetically susceptible label comprising a binding reagent capable of binding the sample compound, and a second label comprising a binding reagent capable of binding the sample compound. The device includes a magnetic field generator configured to subject sample material in the detection zone to a time-varying magnetic field, and a detection system comprising a detector. The detector is configured to receive a time-varying signal from second label associated with the sample compound and a second signal from second label not associated with the sample compound. The detection system is configured to determine the presence of the sample compound based at least in part on the time-varying signal. The device defines an optical path between the detection zone and the detector, the optical path having a spatially varying optical transmittance along an axis normal to the optical path.

The device further includes a light source configured to illuminate sample material within the detection zone. The detector includes a light detector, and the time-varying signal is a time-varying light intensity signal. The detector can be configured to output time-varying response data indicative of the time-varying light intensity signal and the detection system can further include a processor configured to process the time-varying response data to determine the presence of the sample compound. The processor can be configured to autocorrelate the time-varying response data.

The optical path can include a window having a spatially-varying optical transmittance along an axis normal to the optical path, and the light detector can be configured to detect light that has passed through the window from the detection zone. The window can include a periodic spatial optical transmittance.

The second label can be fluorescent at a wavelength emitted by the light source and the light detector can be configured to detect the fluorescence. The binding reagent of the second label can be capable of specifically binding BNP, proBNP, or NTproBNP. The light source can be configured to illuminate contents of the detection zone with a spatially-varying light intensity.

In another aspect, a device for determining a sample compound includes a detection zone configured to accommodate sample material. The detection zone includes a magnetically susceptible label comprising a binding reagent capable of binding the sample compound, and a second label comprising a binding reagent capable of binding the sample compound. The device includes a magnetic field generator configured to subject sample material in the detection zone to a magnetic field, a light source configured to illuminate sample material within the detection zone, and a detection system comprising a light detector. The device defines an optical path between the detection zone and the detector, the optical path having a spatially-varying transmittance along an axis normal to the optical path. The detector can be configured to receive a time-varying optical signal from second label associated with the sample compound and a second optical signal from second label not associated with the sample compound. The detection system is configured to determine the presence of the sample compound based at least in part on the time-varying optical signal. The spatially-varying optical transmittance of the optical path can be periodic. The magnetic field can be an alternating magnetic field.

In another aspect, a method for determining a sample compound includes combining a sample compound, a magnetically susceptible label comprising a binding reagent capable of binding the sample compound, and a second label comprising a binding reagent capable of binding the sample compound. The mixture is subjected to a time-varying magnetic field and illuminated with light. The method includes receiving a time-varying optical signal from second label bound with the sample compound and a second optical signal from second label not bound with the sample compound, and determining the presence of the sample compound based at least in part on the time-varying optical signal.

Detecting can include detecting the time-varying optical signal after the signal has passed along an optical path having a spatially-varying optical transmittance along an axis normal to the optical path. Detecting can include detecting the time-varying optical signal after the signal has passed along the optical path through a window having a spatially-varying transmittance. The window can have a periodic spatially-varying transmittance.

The method can include receiving response data from the detector, the data indicative of the time-varying optical signal and the second optical signal, and determining the presence of the sample compound can include processing the response data. Processing the response data can include autocorrelating the response data. The method can include magnetically moving at least some of the sample material into the detection zone prior to receiving the time-varying optical signal and the second optical signal.

In another aspect, a method includes combining a sample compound, a magnetically susceptible label comprising a binding reagent capable of binding the sample compound, and a second label comprising a binding reagent capable of binding the sample compound. The mixture is subjected to a magnetic field, and illuminated with light to produce a first optical signal from second label bound with the sample compound and a second optical signal from second label not bound with the sample compound. The method includes modulating a temporal frequency of the first optical signal, and determining the presence of the sample compound based at least in part on the first optical signal.

Modulating the temporal frequency can include propagating the first and second optical signals along an optical path having a spatially-varying transmittance along an axis normal to the optical path. The optical path can include a window to the detection zone, the window having a spatially-varying transmittance.

The details of one or more embodiments are set forth in the drawings and description below. Other features, objects, and advantages will be apparent from the description, the drawings, and from the claims.

## BRIEF DESCRIPTION OF THE DRAWINGS

FIG. 1 is a schematic top view of an assay device base.
FIG. 2 is a schematic end view of an assembled assay device.
FIGS. 3A-3B are schematic depictions of reagents and analytes.
FIG. 4A is a schematic view of an assay device in operation. FIG. 4B are depictions of exemplary patterned optical filters.

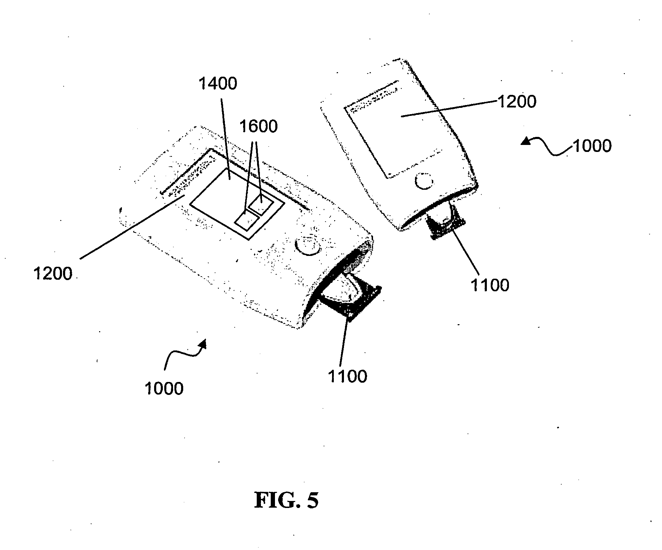

FIG. 5 is an illustration of an assay device reader.

## DETAILED DESCRIPTION

In general, an assay device (e.g., a cartridge or test strip) includes a base and a lid. A void between the base and lid defines a reaction cell which defines the assay volume. The reaction cell is adapted to hold a sample for measurement. The base or lid can have projections that form walls defining the assay volume in an assembled assay device. Alternatively, a third component between the base and lid can provide walls to define the void. The assay device includes a sample inlet that can accept a sample for testing. The sample inlet is fluidly connected by a flow path to the assay volume, so as to deliver a fluid sample from the inlet to the assay volume.

The assay device can include, on a surface of the base, lid, or both, at least one reagent zone, a reference zone, a detection zone, or a combination of these. In some embodiments, the assay device includes a plurality of reagent zones, a reference zone and a detection zone. The reagent zones can overlap with one another or with the reference or detection zones; or the reagent zones can be separated from each other or from the reference and detection zones. The reference and detection zones can be separated from each other, or they can overlap partially or completely. The detection zone and reference zone can be located such that a sample in the assay volume contacts the detection zone and reference zone. A reagent zone can be located such that a sample will contact the reagent zone after the sample is applied to the sample inlet. For example, the reagent zone can be on the flow path, or in the assay volume.

At least one reagent zone includes a first reagent capable of recognizing a desired analyte. Recognition can include binding the analyte. For example, recognition includes selectively binding the analyte; that is, binding the analyte with a higher affinity than other components in the sample. This recognition reagent can be, for example, a protein, a peptide, an antibody, a nucleic acid, a small molecule, a modified antibody, a chimeric antibody; a soluble receptor, an aptamer, or other species capable of binding the analyte. The recognition reagent is optionally linked (e.g., by covalent bond, electrostatic interaction, adsorption, or other chemical or physical linkage) to a reagent that can produce a detectable change. The detectable change can be, for example, a change in optical properties (e.g., a change in absorption, reflectance, refraction, transmittance, or emission of light), or electrical properties (e.g., redox potential, a voltage, a current, or the like).

A reagent zone can include a second reagent capable of recognizing a desired analyte. The second reagent can recognize the same or a different analyte. The first and second recognition reagents can be selected to recognize the same analyte simultaneously. For example the first and second recognition reagents can each be an antibody that recognizes distinct epitopes of the analyte. In this way, a ternary (i.e., three-component) complex of analyte, first recognition reagent and second recognition reagent can be formed. In general, the first and second recognition reagents do not associate with one another in the absence of analyte. The presence of analyte, however, can associate the first and second recognition reagents together, in a ternary complex.

The second recognition reagent can be linked to a surface or to a reagent that can produce a detectable change. The surface can be, for example, a surface of the assay device base, or a surface of a particle. The particle can be, for example, a polymer microsphere, a metal nanoparticle, or a magnetic particle. A magnetic particle is a particle that is influenced by a magnetic field. The magnetic particle can be, for example, a magnetic particle described, in U.S. Patent Application Publication Nos. 20050147963 or 20050100930, or U.S. Patent No. 5,348,876 or commercially available beads, for example, those produced by Dynal AS under the trade name DYNABEADS™. Description of recognition reagents linked to surfaces are described in, for example, U.S. Patent Nos. 6,682,648 and 6,406,913. In particular, antibodies linked to magnetic particles are described in, for example, United States Patent Application Nos. 20050149169, 20050148096, 20050142549, 20050074748, 20050148096, 20050106652, and 20050100930, and U.S. Patent No. 5,348,876.

Generally, the detection zone is the site of a detectable change. The extent of detectable change can be measured at the detection zone. Usually, greater amounts of analyte will result in a greater detectable change; however, the assay can also be configured to produce a smaller change when the analyte is present in greater quantities. The detection zone can optionally be configured to collect the analyte by immobilizing it (for example, with a reagent immobilized in the detection zone, where the immobilized reagent binds to the analyte). Alternatively, the detection zone can optionally be configured to attract or immobilize a component associated with the analyte. For example, a recognition reagent that binds the analyte and is linked to a magnetic particle can be propelled to, or held within, the detection zone by a magnetic field provided in the detection zone. The detection zone can in some embodiments not be configured to

concentrate, confine or immobilize the analyte, but rather simply be the site of the measurement of the detectable change.

In some embodiments, the assay device base, assay device lid, or both have a translucent or transparent window aligned with the detection zone. An optical change that occurs in the detection zone can be detected through the window. Detection can be done visually (i.e., the change is measured by the user's eye) or measured by an instrument (e.g., a photodiode, photomultiplier, or the like).

In general, the reference zone is similar in nature to the detection zone. In other words, when the detection zone includes an electrode, the reference can likewise include an electrode. When the detection zone is aligned with a window for optical measurement, the reference zone can similarly be aligned with a window for optical measurement. The detectable change measured in the reference zone can be considered a background measurement to be accounted for when determining the amount of analyte present in the sample. The reference zone can be identical with the detection zone. In this case, a background measurement can be recorded in the detection zone, usually under some different condition than when the measurement to determine the amount of analyte present in the sample is made.

The sample can be any biological fluid, such as, for example, blood, blood plasma, serum, urine, saliva, tears, or other bodily fluid. The analyte can be any component that is found (or may potentially be found) in the sample, such as, for example, a protein, a peptide, a nucleic acid, a metabolite, a saccharide or polysaccharide, a lipid, a drug or drug metabolite, or other component. For example, the analyte can be a B-type natriuretic peptide (BNP), including but not limited to mature BNP, proBNP, or NTproBNP. The assay device can optionally be supplied with a blood separation membrane arranged between a sample inlet and the detection zone, such that when whole blood is available as a sample, only blood plasma reaches the detection zone.

The assay device and included reagents are typically provided in a dry state. Addition of a liquid sample to the assay device (i.e., to the assay volume) can resuspend dry reagents.

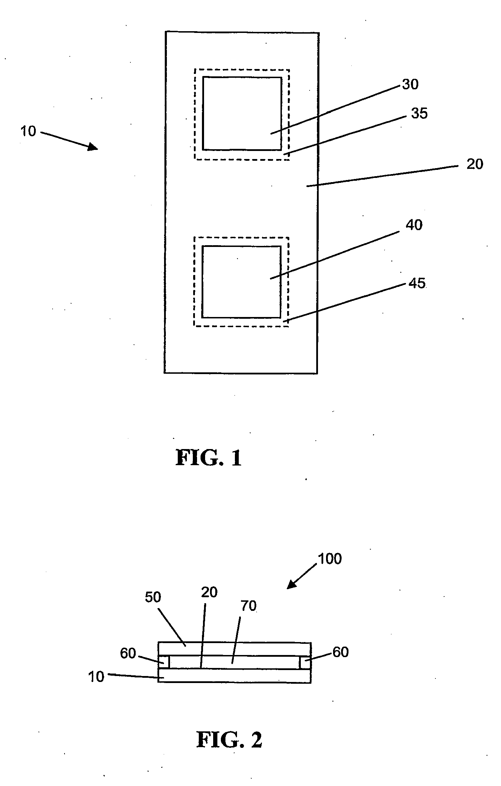

Referring to FIG. 1, assay device base 10 of an assay device includes surface 20. Detection zone 30 and reference zone 40 are disposed on surface 20. First reagent zone 35 overlaps detection zone 30, and second reagent zone 45 overlaps reference zone 40.

Referring to FIG 2, assembled assay device 100 includes base 10 separated from lid 50 by spacers 60. Spacers 60 can be formed as an integral part of base 10 or lid 50. Alternatively, base 10, lid 50 and spacers 60 can be formed separately and assembled together. When assembled, together, connections between base 10, lid 50 and spacers 60 can be sealed, for example with an adhesive or by welding. Base 10, lid 50 and spacers 60 can define a liquid-tight volume 70 where a liquid sample is allowed to contact interior surfaces of volume 70, such as surface 20 of base 10. The dimensions of spacer 60 can be selected such that surfaces of base 10 and lid 50 facing the interior of volume 70 form a capillary, i.e., the base and lid provide capillary action to a liquid inside volume 70. Alternatively, base 10 or lid 50 can provide capillary action independently of each other. Volume 70 can have a volume of less than 100 microliters, less than 20 microliters, less than 10 microliters, or 5 microliters or less.

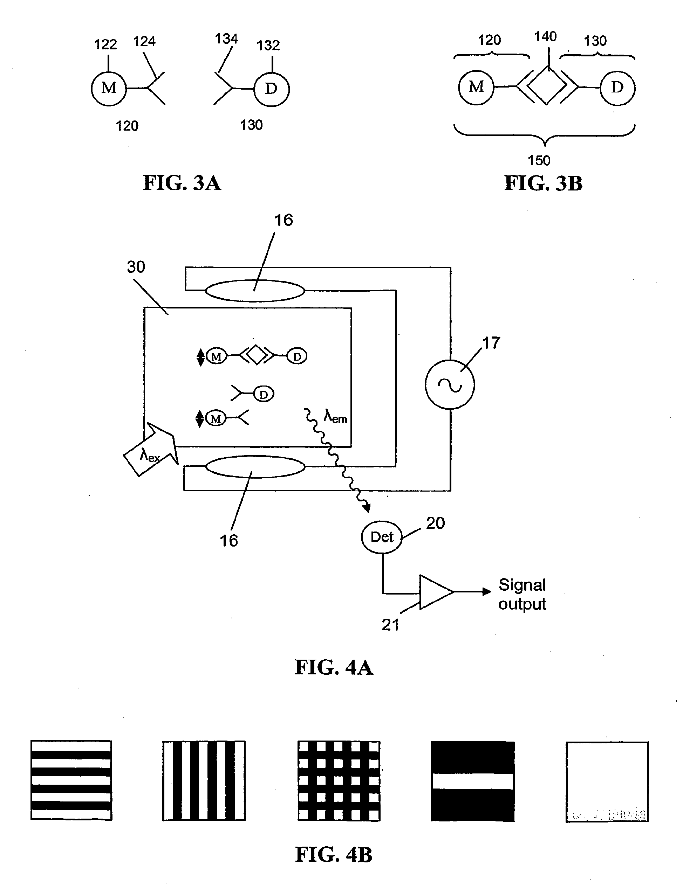

Reagent zone 35 can include reagents 120 and 130, illustrated in FIG. 3A. Reagent 120 includes magnetic particle 122 linked to antibody 124. Reagent 130 includes detectable component 132 linked to antibody 134. Antibodies 124 and 134 are preferably capable of specifically binding to the analyte simultaneously (so that a ternary complex may be formed). When a sample is introduced to volume 70, (for example, by contacting the sample with a sample inlet), liquid can fill volume 70 and contact surface 20 of base 10, resuspending the reagents deposited on surface 20. If the sample contains the analyte recognized by antibodies 124 and 134, then the antibodies will bind to the analyte. The antibodies are chosen to bind to different epitopes of the analyte, allowing the formation of a ternary complex 150 of reagent 120, analyte 140, and reagent 130, as illustrated in FIG. 3B.

FIG. 4A illustrates the assay device, for example, cartridge or test strip, during operation. Detection zone 30 is in contact with liquid sample which includes dissolved reagents and analyte. The reagents can be supplied in excess relative to the amount of analyte present in the sample, such that all analyte is bound, while a portion of the reagents can remain unbound. After the sample is introduced to the assay device, reagents are allowed to contact the sample. For example, when the reagents are initially in a dry state, they can be resuspended by the sample; or when the reagents are initially in a liquid solution, they can be mixed with a liquid sample. Reagents, analytes, and complexes can be distributed by diffusion near the location in volume 70. Magnetic field source 160 is located proximate to detection zone 30.

Magnetic field source 160 can include, for example, a permanent magnet or an electromagnet. The magnetic field source 160 can be a single magnet (e.g., one permanent magnet, or one electromagnet) or can include more than one magnets. Magnetic field source 160 can be configured to apply a time-varying magnetic field to detection zone 30. In one embodiment, the time-varying magnetic field is applied by supplying a time-varying current to one or more electromagnets. In another embodiment, a permanent magnet can be moved in a time-varying manner relative to detection zone 30. In some embodiments, the time-variations of the magnetic field are periodic and can be described by a waveform, such as, for example, a sine wave, square wave, triangular wave, or any other desired waveform.

FIG. 4A schematically illustrates an embodiment where magnetic field source 160 includes electromagnets electrically connected to waveform generator 170. Variations in the magnetic field can induce reagent 120 to move, as magnetic forces act on magnetic particles 122. When the time variations in the magnetic field are periodic, the movement of reagent 120 can likewise be periodic, and have a period matching that of the applied waveform. When the analyte is present, a fraction of detectable reagent 130 will be bound in ternary complexes 150, and the remaining fraction of detectable reagent 130 remains unbound (or free). Ternary complexes 150 are also susceptible to variations in magnetic field, and the ternary complexes can also move with a period matching that of the applied waveform. The unbound fraction of detectable reagent 130 is not susceptible to magnetic forces and will not move with the same period as the applied waveform.

Detectable component 132 can be directly detectable, or component 132 can be detected indirectly. Component 132 can produce a product that is directly detected, such that detection of the product is an indirect detection of component 132. For example, component 132 can be an enzyme whose product is detected directly (e.g., optically or electrochemically). The amount of product formed, or rate of product formation, can be related to the amount of detectable component 132. Optical properties of component 132 can be directly detected. For example, component 132 can be a colored particle detected by observation of a color change, or change in intensity of color (i.e., absorbance or optical density). Component 132 can be a fluorescent particle or chemiluminescent particle detectable by observation of light emission.

FIG. 4A illustrates an embodiment where detectable component 132 is a fluorescent particle. Fluorescent emission from the particle is produced when an excitation wavelength of light ($\lambda_{ex}$) is absorbed by the fluorescent particle, followed by emission of an emission wavelength of light ($\lambda_{em}$). The emitted light can travel along an optical path to a detector. The optical path can include optical elements to modify light that reaches detector 200. Detector 200 can be, for example, a photodiode, or other element that produces an electrical signal which is related in some property (e.g., current) to the intensity of light reaching the detector. The electrical signal produced by detector 200 can be modified by signal processor 210. The optical components can include, for example, a lens (e.g., an objective lens to collect light; or a lens to focus light on the detector), a filter (such as, for example, a cut-off filter to block scattered excitation light). The optical path can include a patterned filter. The patterned filter can have a spatially-varying transmittance along on axis normal to the optical path. For example, the patterned filter can include a pattern of stripes, where alternating stripes have lower transmittance (in particular, to the emission wavelength) than neighboring stripes. Some examples of suitable patterns are illustrated in FIG. 4B, and include stripes, squares (e.g., a checkerboard pattern), a slit or single stripe, or a graded scale. The regions of lower transmittance can reduce transmittance (compared to regions of higher transmittance) by at least 5%, at least 10%, at least 25%, at least 50%, at least 75%, at least 90%, at least 95%, or more. The regions of lower transmittance can be substantially opaque.

The magnetic field source and patterned filter can be oriented so that the time-varying magnetic field will move the magnetic particles relative to the pattern. When the analyte is present in the sample, a fraction of the magnetic particles can be included in a ternary complex with a fluorescent particle. As the particles move, regions of low transmittance will come between the particle and the ternary complexes. When the ternary complex moves such that a region of low transmittance is between the particle and the detector, the detector records a decrease in light detected. When the ternary complex moves such that a region of high transmittance is between the particle and the detector, the detector records an increase in light detected. When the time-varying magnetic field is periodic, the motion of the particles can also be periodic. The frequency of the periodic motion of the particles can be different than the frequency of the time variations in the magnetic field. The detector will record periodic changes in light detected. The frequency of the optical signal does not necessarily match the frequency of the magnetic field. The frequency of the optical signal can be influenced by the spatial period of the patterned filter over the detection window and the velocity at which the particles move relative to the patterned filter. The particle velocity can be related to the strength of the magnetic field and other factors. The fraction of fluorescent particles that remain unbound to magnetic particles do not move in response to the time-varying magnetic field, and detector records a time-invariant emission of light from this fraction of fluorescent particles.

Signal processor 210 can include an electronic bandpass filter. An electronic bandpass filter is an electronic device that accepts an electronic signal and allows only periodic signals of a predetermined frequency (or range of frequencies) to pass through the filter as a signal output. When the bandpass filter is configured to allow only signals having a period matching that of periodic motion of the ternary complexes, the electronic bandpass filter can be used to distinguish the signal from detectable particles that are part of a ternary complex from the unbound detectable particles.

Properties of the waveform (including period, intensity, shape and duration) can be selected based on the fluid properties of the sample fluid, such as, for example, viscosity, temperature, and surface tension. The size and geometry of the assay volume can also influence the choice of waveform. If the desired motion of the magnetic particles is a periodic motion, the waveform properties can be chosen such that magnetic particles suspended in the sample fluid will respond to the applied time-varying magnetic field with a periodic motion. Selection of the appropriate waveform can also be influenced by an expected range of properties for a type of sample. For example, the viscosity of saliva samples may vary from sample to sample, and the waveform can be chosen for its ability to induce the desired particle motion for such a range of fluid properties.

The detector can be used to take a first and a second measurement. The first measurement can be recorded while a time-varying magnetic field is applied. The first measurement records light emitted from particles that are part of a ternary complex. The second measurement is recorded while the magnetic field is not changing with time (e.g., zero applied field or constant applied field). The second measurement will therefore detect light emitted from all detectable particles, whether or not they are part of a ternary complex. When a patterned optical filter and an electronic bandpass filter are in use, the electronic bandpass filter can operate only on the measurement recorded while the time-varying magnetic field is applied.

The ratio between the first and second measurements is related to the fraction of detectable particles that are part of a ternary complex. The fraction of detectable particles that are part of a ternary complex is, in turn, related to the concentration of analyte in the sample. Thus, the two measurements can be used to calculate a concentration of analyte in the sample.

Another method for distinguishing the bound and free fractions of detectable particles involves a correlation measurement. The correlation method can operate without a patterned filter in the optical path or an electronic bandpass filter on the detector output signal. The correlation method takes advantage of the fact that a detectable particle will move at a substantially different rate depending on whether it is bound to a magnetic particle or remains free. As such, if mechanical fluid motion (induced by the motion of the magnetic particles in a time-varying magnetic field) induces motion of free detectable particles, the free and bound particles can still be distinguished.

The correlation method begins by measuring a detectable signal (e.g., fluorescence) from the detectable particles. Typically, the signal is measured in a defined region of fluid in which the detectable particles are moving. The motion can be simple diffusion or can be motion induced by an applied force, such as the force generated by a magnetic field acting on a magnetic particle. The signal is recorded continuously for a period of time substantially longer than the time required for a slow-moving particle to cross the defined region. In the case of a fluorescent signal, the defined region can be continuously illuminated with an excitation wavelength. For example, the signal can be recorded continuously for one second or longer, such as five seconds or longer, ten seconds or longer, or one minute or longer. Because the correlation method relies on the statistical behavior of the detectable particles, the length of time for which a signal is recorded can be selected to ensure that an adequate number of particles are recorded for a reliable measurement.

While the signal is being recorded, particles will move into and out of the defined region. The motion can be due to simple diffusion, fluid flow, an applied magnetic field acting on magnetic particles, a combination of these, or other forces. Larger particles (e.g., detectable particles bound to a magnetic particle via an analyte) move more slowly than smaller particles. The recorded fluorescence intensity will fluctuate as a function of time depending on factors including the excited state lifetime of the fluorescent species, and the entrance and exit of fluorescent particles from the defined region. The recorded fluorescence intensity as a function of time typically appears to be a noisy signal. Statistically, the number of detectable particles in the defined region at a given time can be described by a Poisson distribution.

The correlation analysis involves determining the product of the recorded signal F(t) with itself (i.e., autocorrelation), offset by a time differential, $\tau$. In one embodiment, a normalized autocorrelation function $G(\tau)$ of the time-varying fluorescence signal $F(t)$ can be defined as:

$$G(\tau) = \frac{\langle \partial F(t) \cdot \partial F(t+\tau) \rangle}{\langle F(t) \rangle^2}.$$

Other autocorrelation functions are known. The autocorrelation function can also be related to the time constants of free and bound label, $\tau_{free}$ and $\tau_{bound}$, respectively and the fraction of bound label. See, for example, M. Kinjo and R. Rigler, Nucl. Acids Res. 1995, 23, 1795-1799. In that work, a fluorescence autocorrelation function was used to determine the fraction of a fluorescently labeled 18-mer DNA bound to a 7.5 kb DNA. Briefly, an equation was developed that described $G(\tau)$ in terms of bound fraction, $\tau_{free}$ and $\tau_{bound}$, and a parameter related to the size of the defined region. The experimental data was fit to that equation using a non-linear least squares method. See also R. Rigler and E. L. Elson, Fluorescence Correlation Spectroscopy: Theory and Applications (Springer, Berlin, 2001); and Oleg Krichevsky et al. 2002 Rep. Prog. Phys. 65 251-297. In some circumstances, the value of one or more parameters used to fit $G(\tau)$ can be fixed to a predetermined value. For example, the value of $\tau_{free}$ can be determined experimentally ahead of time, and be held at that fixed value when fitting the $G(\tau)$ model to the experimental data. Fluorescence correlation can also be performed with more than one distinct fluorescent label (e.g., two or more fluorescent species each having distinct emission wavelengths). See, e.g., Schwille, P. et al., Biophys. J. 1997, 72, 1878-1886.

In implementing a correlation method for analyte detection, the signal can be sent to a processor programmed with instructions for recording a signal from a detector, applying the correlation analysis, and calculating a result for the fraction of bound particles. The processor can also be programmed to calculate the concentration of analyte in the sample based on the fraction of bound particles. The processor can be further programmed with instructions for displaying, storing, or transmitting the recorded signal and calculated results. For example, the processor can be programmed to display a calculated analyte concentration on a display screen; to store the calculated analyte concentration in a memory; or to transmit the recorded signal or calculated analyte concentration to a second processor for further calculation or storage.

The first and second measurements can be repeated a number of times. For example, the first measurement can be repeated with different frequencies for the time-varying magnetic field. Repeated measurements allow the concentration of analyte in the sample to be measured with higher confidence than if only a single measurement was used.

Detectable component 132 can be selected to produce an optical change. For example, a detectable change in chemiluminescent signal can be produced by a chemical reaction involving a soluble component and a component fixed to a particle. The chemical reaction results in a detectable emission of light. In some embodiments, the chemiluminscent signal is produced only when an analyte molecule in a sample brings two particles (or beads) together in close proximity. A first particle, called a donor particle, is linked to a first antibody, and a second particle (an acceptor particle) is linked to a second antibody. The first and second antibodies bind to different epitopes of the same antigen, such that a ternary complex of donor particle-antigen-acceptor particle can be formed. A cascade of chemical reactions that depends on the proximity of the beads (and therefore on the presence of the analyte) can produce greatly amplified signal. Detection of an analyte at attomolar (i.e., on the order of $10^{-18}$ molar) concentrations is possible.

Photosensitizer particles (donor particles) including a phthalocyanine can generate singlet oxygen when irradiated with light having a wavelength of 680 nm. The singlet oxygen produced has a very short half-life - about 4 microseconds - and hence it decays rapidly to a ground state. Because of the short half-life, singlet oxygen can only diffuse to a distance of a few hundred microns from the surface of the particles before it decays to ground state. The singlet state survives long enough, however, to enter a second particle held in close proximity. The second particles (acceptor particles) include a dye that is activated by singlet oxygen to produce chemiluminescent emission. This chemiluminescent emission can activate further fluorophores contained in the same particle, subsequently causing emission of light at 520-620 nm. See, for example, Proc. Natl. Acad. Sci. 91:5426-5430 1994; and U.S. Patent No. 6,143,514.

An optical change can also be produced by a bead linked to an antibody. The bead can include a polymeric material, for example, latex or polystyrene. To produce the optical change, the bead can include a light-absorbing or light-emitting compound. For example, a latex bead can include a dye or a fluorescent compound. The reagent can include a plurality of beads. The beads in the plurality can be linked to one or more distinct antibodies. A single bead can be linked to two or more distinct antibodies, or each bead can have only one distinct antibody linked to it. The reagent can have more than one distinct antibody each capable of binding to the same analyte, or antibodies that recognizes different analytes. When the bead includes a light absorbing compound, the optical measurement can be a measurement of transmittance, absorbance or reflectance. With a fluorescent compound, the intensity of emitted light can be measured. The extent of the measured optical change can be correlated to the concentration of analyte in the sample.

A detectable change can be produced by the enzyme multiplied immunoassay technique (EMIT). In an EMIT assay format, an enzyme-analyte conjugate is used. A first reagent can include an antibody specific for the analyte, an enzyme substrate, and (optionally) a coenzyme. A second reagent can include a labeled analyte: a modified analyte that is linked to an enzyme. For example, the enzyme can be a glucose-6-phosphate dehydrogenase (G-6-PDH). G-6-PDH can catalyze the reaction of glucose-6-phosphate with NAD(P) to yield 6-phosphoglucono-D-lactone and NAD(P)H. NAD(P)H absorbs light with a wavelength of 340 nm, whereas NAD(P) does not. Thus, a change in absorption of 340 nm light as a result of the G-6-PDH catalyzed reaction can be a detectable change. When the first reagent is mixed with a sample, the analyte is bound by the antibody in the first reagent. The second reagent is added, and any free antibody binding sites are occupied by the enzyme-linked analyte of the second reagent. Any remaining free antibodies bind the labeled analyte, inactivating the linked enzyme. Labeled analyte bound by the antibody is inactive, i.e., it does not contribute to the detectable change. Labeled analyte that is not bound by antibody (a quantity proportional to amount of analyte in sample) reacts with the substrate to form a detectable product (e.g., NAD(P)H).

Another assay format is the cloned enzyme donor immunoassay (CEDIA). CEDIA is a homogeneous immunoassay based on the bacterial enzyme β-galactosidase of *E. coli* which has been genetically engineered into two inactive fragments. These two inactive fragments can recombine to form an active enzyme. One fragment consists of an analyte-fragment conjugate, and the other consists of an antibody-fragment conjugate. The amount of active enzyme that generates the signal is proportional to the analyte concentration. See, for example, Khanna, P.L. and Coty, W.A. (1993) In: Methods of Immunological Analysis, volume 1 (Masseyeff, R.F., Albert, W.H., and Staines, N.A., eds.) Weinheim, FRG: VCH Verlagsgesellschaft MbH, 1993: 416-426; Coty, W.A., Loor, R., Powell, M., and Khanna, P.L. (1994) J. Clin. Immunoassay 17(3): 144-150; and Coty, W.A., Shindelman, J., Rouhani, R. and Powell, M.J. (1999) Genetic Engineering News 19(7).

The assay device can be used in combination with a reader configured to measure the detectable change. The reader can include an optical system to detect light from the analysis region. The light to be detected can be, for example, emitted, transmitted, reflected, or scattered from the detection zone. Emitted light can result from, for example, chemiluminescent or fluorescent emission. The optical system can include an illumination source, for example, to be used in the detection of a change in fluorescence, absorbance, or reflection of light. For an assay device configured for an electrochemical measurement, the reader can be in electrical contact with the working electrode and reference electrode. The assay device electrodes can have electrical leads connecting the electrodes to contacts outside the assay void. The contacts register with and contact corresponding contacts of the assay device to provide electrical contact. The reader can also include an output display configured to display the results of the measurement to a user.

The assay device reader can include magnetic field source 160. The assay device reader can be configured to apply a magnetic field via source 160 at predetermined times, such as after a predetermined period of time has elapsed after a sample has been applied to the assay device. Magnetic field source 160 can be, for example, an electromagnet or a permanent magnet. An electromagnet can selectively apply a field when a current is supplied to the electromagnet. A permanent magnet can be moved toward or away from the detection zone in order to control the strength of the field at that site. The permanent magnet can be moved in a period fashion to create a time-varying magnetic field. The assay device reader can include a waveform generator for applying a desired time-varying magnetic field. The assay device reader can also include any optics (e.g., illumination sources, lenses, optical filters, optical detectors, and the like) needed to perform a measurement. The reader can also include electronics for signal processing (including, for example, an electronic bandpass filter) and a processor for performing predetermined calculations and storage.

Referring to FIG. 6, reader instrument 1000 accepts test assay device 1100 and includes display 1200. The display 1200 may be used to display images in various formats, for example, text, joint photographic experts group (JPEG) format, tagged image file format (TIFF), graphics interchange format (GIF), or bitmap. Display 1200 can also be used to display text messages, help messages, instructions, queries, test results, and various information to patients.

Display 1200 can provide a user with an input region 1400. Input region 1400 can include keys 1600. In one embodiment, input region 1400 can be implemented as symbols displayed on the display 1200, for example when display 1200 is a touch-sensitive screen. User instructions and queries are presented to the user on display 1200. The user can respond to the queries via the input region.

Reader 1000 also includes an assay device reader, which accepts diagnostic test assay devices 1100 for reading. The assay device reader can measure the level of an analyte based on, for example, the magnitude of an optical change, an electrical change, or other detectable change that occurs on a test assay device 1100. For reading assay devices that produce an optical change in response to analyte, the assay device reader can include optical systems for measuring the detectable change, for example, a light source, filter, and photon detector, e.g., a photodiode, photomultiplier, or Avalanche photo diode. For reading assay devices that produce an electrical change in response to analyte, the assay device reader can include electrical systems for measuring the detectable change, including, for example, a voltameter or amperometer.

Device 1000 further can include a communication port (not pictured). The communication port can be, for example, a connection to a telephone line or computer network. Device 1000 can communicate the results of a measurement to an output device, remote computer, or to a health care provider from a remote location.

A patient, health care provider, or other user can use reader 1000 for testing and recording the levels of various analytes, such as, for example, a biomarker, a metabolite, or a drug of abuse. Various implementations of diagnostic device 1000 may access programs and/or data stored on a storage medium (e.g., a hard disk drive (HDD), flash memory, video cassette recorder (VCR) tape or digital video disc (DVD); compact disc (CD); or floppy disk). Additionally, various implementations may access programs and/or data accessed stored on another computer system through a communication medium including a direct cable connection, a computer network, a wireless network, a satellite network, or the like.

The software controlling the reader can be in the form of a software application running on any processing device, such as, a general-purpose computing device, a personal digital assistant (PDA), a special-purpose computing device, a laptop computer, a handheld computer, or a network appliance.

The reader may be implemented using a hardware configuration including a processor, one or more input devices, one or more output devices, a computer-readable medium, and a computer memory device. The processor may be implemented using any computer processing device, such as, a general-purpose microprocessor or microcontroller or an application-specific integrated circuit (ASIC). The processor can be integrated with input/output (I/O) devices to provide a mechanism to receive sensor data and/or input data and to provide a mechanism to display or otherwise output queries and results to a service technician. Input device may include, for example, one or more of the following: a mouse, a keyboard, a touch-screen display, a button, a sensor, and a counter.

The display 1200 may be implemented using any output technology, including a liquid crystal display (LCD), a television, a printer, and a light emitting diode (LED). The computer-readable medium provides a mechanism for storing programs and data either on a fixed or removable medium. The computer-readable medium may be implemented using a conventional computer hard drive, or other removable medium. Finally, the system uses a computer memory device, such as a random access memory (RAM), to assist in operating the reader.

Implementations of the reader can include software that directs the user in using the device, stores the results of measurements. The reader 1000 can provide access to applications such as a medical records database or other systems used in the care of patients. In one example, the device connects to a medical records database via the communication port. Device 1000 may also have the ability to go online, integrating existing databases and linking other websites.

In general, the assay device can be made by depositing reagents on a base and sealing a lid over the base. The base can be a micro-molded platform or a laminate platform.

Micro-molded platform

For an assay device prepared for optical detection, the base, the lid, or both base and lid can be transparent to a desired wavelength of light. Typically both base and lid are transparent to visible wavelengths of light, e.g., 400-700 nm. The base and lid can be transparent to UV and near IR wavelengths, for example, to provide a range of wavelengths that can be used for detection, such as 200 nm to 1000 nm, or 300 nm to 900 nm.

For an assay device that will use electrochemical detection, electrodes are deposited on a surface of the base. The electrodes can be deposited by screen printing on the base with a carbon or silver ink, followed by an insulation ink; by evaporation or sputtering of a conductive material (such as, for example, gold, silver or aluminum) on the base, followed by laser ablation; or evaporation or sputtering of a conductive material (such as, for example, gold, silver or aluminum) on the base, followed by photolithographic masking and a wet or dry etch.

An electrode can be formed on the lid in one of two ways. A rigid lid can be prepared with one or more through holes, mounted to a vacuum base, and screen printing used to deposit carbon or silver ink. Drawing a vacuum on the underside of the rigid lid while screen printing draws the conductive ink into the through holes, creating electrical contact between the topside and underside of the lid, and sealing the hole to ensure that no liquid can leak out. Alternatively, the lid can be manufactured without any through holes and placed, inverted, on a screen printing platform, where carbon or silver ink is printed.

Once the electrodes have been prepared, the micro-molded bases are loaded and registered to a known location for reagent deposition. Deposition of reagents can be accomplished by dispensing or aspirating from a nozzle, using an electromagnetic valve and servo- or stepper-driven syringe. These methods can deposit droplets or lines of reagents in a contact or non-contact mode. Other methods for depositing reagents include pad printing, screen printing, piezoelectric print head (e.g., ink-jet printing), or depositing from a pouch which is compressed to release reagent (a "cake icer"). Deposition can preferably be performed in a humidity- and temperature-controlled environment. Different reagents can be dispensed at the same or at a different station.

Fluorescent or colored additives can optionally be added to the reagents to allow detection of cross contamination or overspill of the reagents outside the desired deposition zone. Product performance can be impaired by cross-contamination. Deposition zones can be in close proximity or a distance apart. The fluorescent or colored additives are selected so as not to interfere with the operation of the assay device, particularly with detection of the analyte.

After deposition, the reagents are dried. Drying can be achieved by ambient air drying, infrared drying, infrared drying assisted by forced air, ultraviolet light drying, forced warm, controlled relative humidity drying, or a combination of these.

Micro-molded bases can then be lidded by bonding a flexible or rigid lid on top. Registration of the base and lid occurs before the two are bonded together. The base and lid can be bonded by heat sealing (using a heat activated adhesive previously applied to lid or base, by ultrasonic welding to join two similar materials, by laser welding (mask or line laser to join two similar materials), by cyanoacrylate adhesive, by epoxy adhesive previously applied to the lid or base, or by a pressure sensitive adhesive previously applied to the lid or base.

After lidding, some or all of the assembled assay devices can be inspected for critical dimensions, to ensure that the assay device will perform as designed. Inspection can include visual inspection, laser inspection, contact measurement, or a combination of these.

The assay device can include a buffer pouch. The buffer pouch can be a molded well having a bottom and a top opening. The lower opening can be sealed with a rupturable foil or plastic, and the well filled with buffer. A stronger foil or laminate is then sealed over the top opening. Alternatively, a preformed blister pouch filled with buffer is placed in and bonded in the well. The blister pouch can include 50 to 200 $\mu$L of buffer and is formed, filled, and sealed using standard blister methods. The blister material can be foil or plastic. The blister can be bonded to the well with pressure sensitive adhesive or a cyanoacrylate adhesive.

Laminate platform

Three or more laminates, fed on a roll form at a specified width, can be used to construct an assay device. The base laminate is a plastic material and is coated on one surface with a hydrophilic material. This laminate is fed into a printing station for deposition of conductive electrodes and insulation inks. The base laminate is registered (cross web) and the conductive electrodes deposited on the hydrophilic surface, by the techniques described previously.

The base laminate is then fed to a deposition station and one or more reagents applied to the laminate. Registration, both cross web and down web, occurs before reagents are deposited by the methods described above. The reagents are dried following deposition by the methods described above.

A middle laminate is fed in roll form at a specified width. There can be more than one middle laminate in an assay device. The term middle serves to indicate that it is not a base laminate or lid laminate. A middle laminate can be a plastic spacer with either a pressure sensitive adhesive or a heat seal adhesive on either face of the laminate. A pressure sensitive adhesive is provided with a protective liner on either side to protect the adhesive. Variations in the thickness of the middle laminate and its adhesives is less than 15%, or less than 10%.

Channels and features are cut into the middle laminate using a laser source (e.g., a $CO_2$ laser, a YAG laser, an excimer laser, or other). Channels and features can be cut all the way through the thickness of the middle laminate, or the features and channels can be ablated to a controlled depth from one face of the laminate.

The middle and base laminates are registered in both the cross web and down web directions, and bonded together. If a pressure sensitive adhesive is used, the lower liner is removed from the middle laminate and pressure is applied to bond the base to the middle laminate. If a heat seal adhesive is used, the base and middle laminate are bonded using heat and pressure.

The top laminate, which forms the lid of the assay device, is fed in roll form at a specified width. The top laminate can be a plastic material. Features can be cut into the top laminate using a laser source as described above. The top laminate is registered (cross web and down web) to the base and middle laminates, and bonded by pressure lamination or by heat and pressure lamination, depending on the adhesive used.

After the laminate is registered in cross and down web directions, discrete assay devices or test strips are cut from the laminate using a high powered laser (such as, for example, a $CO_2$ laser, a YAG laser, an excimer laser, or other).

Some or all of the assembled assay devices can be inspected for critical dimensions, to ensure that the assay device will fit perform as designed. Inspection can include visual inspection, laser inspection, contact measurement, or a combination of these.

Other embodiments are within the scope of the following claims.

**Claims**

1.  A device for determining a sample compound, comprising: a detection zone configured to accommodate sample material comprising: a magnetically susceptible label comprising a binding reagent capable of binding the sample compound, and a second label comprising a binding reagent capable of binding the sample compound, a magnetic field generator configured to subject sample material in the detection zone to a magnetic field, a light source configured to illuminate sample material within the detection zone, a detection system comprising a light detector; wherein: the device defines an optical path between the detection zone and the detector, the optical path having a spatially- varying transmittance along an axis normal to the optical path, the detector is configured to receive a time-varying optical signal comprising a first optical signal from second label associated with the sample compound and a second optical signal from second label not associated with the sample compound, and the detection system is configured to determine the presence of the sample compound based at least in part on the time-varying optical signal.

2.  The device of claim 1, wherein the spatially-varying optical transmittance of the optical path is periodic.

3.  The device of claim 1 or 2, wherein the magnetic field is an alternating magnetic field.

4.  The device of claim 1, wherein the magnetic field generator is configured to subject sample material in the detection zone to a time- varying magnetic field.

5.  The device of claim 1, 2, 3 or 4, wherein the detector is configured to output time-varying response data indicative of the time-varying optical signal and the detection system further includes a processor configured to process the time-varying response data to determine the presence of the sample compound and wherein the processor is configured to autocorrelate the time-varying response data and to determine the presence of the sample compound based at least in part on a portion of the autocorrelated data that is indicative of the first signal.

6.  The device of claim 5, wherein the detection system includes an electronic filter configured to receive the time- varying response data, the electronic filter having a frequency response configured to pass frequencies corresponding to the first signal and to relatively reject frequencies corresponding to the second signal.

7.  The device of any one of claims 1 to 6, wherein the optical path comprises a window having a spatially-varying optical transmittance along an axis normal to the optical path and the light detector is configured to detect light that has passed through the window from the detection zone, or wherein the window comprises a periodic spatial optical transmittance.

8.  The device of any one of claims 1 to 7, wherein second label is fluorescent upon illumination with a wavelength emitted by the light source and the light detector is configured to detect the fluorescence and wherein the binding reagent of the second label is capable of specifically binding BNP, proBNP, or NT-proBNP.

9.  A method, comprising: combining a sample compound, a magnetically susceptible label comprising a binding reagent capable of binding the sample compound, and a second label comprising a binding reagent capable of binding the sample compound, subjecting the mixture to a time- varying magnetic field, illuminating the mixture with light, receiving a time- varying optical signal comprising a first optical signal from second label bound with the sample compound and a second optical signal from second label not bound with the sample compound, and determining the presence of the sample compound based at least in part on the time- varying optical signal, the method further comprising magnetically moving at least some of the sample material into the detection zone prior to receiving the time- varying optical signal and the second optical signal.

10.  The method of claim 9, wherein detecting comprises detecting the time- varying optical signal after the signal has passed along an optical path having a spatially- varying optical transmittance along an axis normal to the optical path, or wherein detecting comprises detecting the time- varying optical signal after the signal has passed along the optical path through a window having a spatially- varying transmittance, and wherein the window has a periodic spatially- varying transmittance.

11.  The method of claim 9 or 10, further comprising receiving response data from the detector, the data indicative of the time- varying optical signal and the second optical signal, and determining the presence of the sample compound comprises processing the response data.

12.  The method of any one of claims 9 to 11, wherein processing the response data comprises autocorrelating the response data and determining the presence of the sample compound based at least in part on a portion of the autocorrelated data that is indicative of the first signal.

13. A method, comprising: combining a sample compound, a magnetically susceptible label comprising a binding reagent capable of binding the sample compound, and a second label comprising a binding reagent capable of binding the sample compound, subjecting the mixture to a magnetic field, illuminating the mixture with light to produce a first optical signal from second label bound with the sample compound and a second optical signal from second label not bound with the sample compound, modulating a temporal frequency of the first optical signal, and determining the presence of the sample compound based at least in part on the first optical signal.

14. The method of claim 13, wherein modulating the temporal frequency comprises propagating the first and second optical signals along an optical path having a spatially-varying transmittance along an axis normal to the optical path, wherein the optical path comprises a window to the detection zone, the window having a spatially- varying transmittance.

**Patentansprüche**

1. Vorrichtung zum Bestimmen einer Probenverbindung, umfassend: eine Erfassungszone, die ausgestaltet ist, um ein Probenmaterial aufzunehmen, umfassend: eine magnetempfindliche Kennzeichnung, die ein Bindungsreagens umfasst, das imstande ist die Probenverbindung zu binden, und eine zweite Kennzeichnung, die ein Bindungsreagens umfasst, das imstande ist die Probenverbindung zu binden, einen Magnetfelderzeuger, der ausgestaltet ist, um Probenmaterial in der Erfassungszone einem Magnetfeld auszusetzen, eine Lichtquelle, die ausgestaltet ist. Probenmaterial innerhalb der Erfassungszone zu beleuchten, ein Erfassungssystem mit einem Lichtdetektor; wobei: die Vorrichtung einen Lichtweg zwischen der Erfassungszone und dem Detektor definiert, der Lichtweg eine räumlich-variierende Transmission entlang einer Achse senkrecht zu dem Lichtweg aufweist, der Detektor ausgestaltet ist, um ein zeitlich-variierendes optisches Signal zu empfangen, umfassend ein erstes optisches Signal von der zweiten Kennzeichnung, die mit der Probenverbindung assoziiert ist, und ein zweites optisches Signal von der zweiten Kennzeichnung, die nicht mit der Probenverbindung assoziiert ist, und das Erfassungssystem ausgestaltet ist, um das Vorhandensein der Probenverbindung basierend zumindest teilweise auf dem zeitlich-variierenden optischen Signal zu bestimmen.

2. Vorrichtung nach Anspruch 1, wobei die räumlich-variierende optische Transmission des Lichtwegs periodisch ist.

3. Vorrichtung nach Anspruch 1 oder 2, wobei das Magnetfeld ein Wechselmagnetfeld ist.

4. Vorrichtung nach Anspruch 1, wobei der Magnetfeldgenerator ausgestaltet ist, um Probenmaterial in der Erfassungszone einem zeitlich-variierenden Magnetfeld auszusetzen.

5. Vorrichtung nach Anspruch 1, 2, 3 oder 4, wobei der Detektor ausgestaltet ist, um zeitlich-variierende Antwortdaten auszugeben, die auf das zeitlich-variierende optische Signal hindeuten, und das Erfassungssystem ferner einen Prozessor umfasst, der ausgestaltet ist, um die zeitlich-variierenden Antwortdaten zu verarbeiten, um das Vorhandensein der Probenverbindung zu bestimmen, und wobei der Prozessor ausgestaltet ist, um die zeitlich-variierenden Antwortdaten automatisch zu korrelieren und das Vorhandensein der Probenverbindung zu bestimmen, basierend zumindest teilweise auf einem Teil der automatisch korrelierten Daten, die auf das erste Signal hindeuten.

6. Vorrichtung nach Anspruch 5, wobei das Erfassungssystem einen elektronischen Filter umfasst, der ausgestaltet ist, um die zeitlich-variierenden Antwortdaten zu empfangen, der elektronische Filter ein Frequenzverhalten aufweist, das ausgestaltet ist, um Frequenzen durchzulassen, die dem ersten Signal entsprechen, und Frequenzen relativ abzuweisen, die dem zweiten Signal entsprechen.

7. Vorrichtung nach einem der Ansprüche 1 bis 6, wobei der Lichtweg ein Fenster umfasst, das eine räumlich-variierende optische Transmission entlang einer Achse senkrecht zu dem Lichtweg aufweist, und der Lichtdetektor ausgestaltet ist, um Licht zu erfassen, das durch das Fenster von der Erfassungszone durchgegangen ist, oder wobei das Fenster eine periodische räumliche optische Transmission aufweist.

8. Vorrichtung nach einem der Ansprüche 1 bis 7, wobei die zweite Kennzeichnung bei Beleuchtung mit einer durch die Lichtquelle emittierten Wellenlänge fluoreszierend ist und der Lichtdetektor ausgestaltet ist, um die Fluoreszenz zu erfassen, und wobei das Bindungsreagens der zweiten Kennzeichnung imstande ist, spezifisch BNP, proBNP oder NTproBNT zu binden.

9. Verfahren, umfassend: Kombinieren einer Probenverbindung, einer magnetempfindlichen Kennzeichnung, umfassend ein Bindungsreagens, das imstande ist die Probenverbindung zu binden, und einer zweiten Kennzeichnung, umfassend ein Bindungsreagens, das imstande ist die Probenverbindung zu binden, Aussetzen der Mischung an ein zeitlich-variierendes Magnetfeld, Beleuchten der Mischung mit Licht, Empfangen eines zeitlich-variierenden optischen Signals, umfassend ein erstes optisches Signal von der zweiten Kennzeichnung, die mit der Probenverbindung verbunden ist, und ein zweites optisches Signal von der zweiten Kennzeichnung, die nicht mit der Probenverbindung verbunden ist, und Bestimmen des Vorhandenseins der Probenverbindung basierend zumindest teilweise auf dem zeitlich-variierenden optischen Signal, wobei das Verfahren ferner ein magnetisches Bewegen von zumindest etwas des Probenmaterials in die Erfassungszone vor dem Empfangen des zeitlich-variierenden optischen Signals und des zweiten optischen Signals umfasst.

10. Verfahren nach Anspruch 9, wobei das Erfassen ein Erfassen des zeitlich-variierenden optischen Signals umfasst, nachdem das Signal entlang eines Lichtwegs mit einer räumlich-variierenden optischen Transmission entlang einer Achse senkrecht zu dem Lichtweg durchging, oder wobei das Erfassen ein Erfassen des zeitlich-variierenden optischen Signals umfasst, nachdem das Signal entlang dem Lichtweg durch ein Fenster mit einer räumlich-variierenden Transmission durchging, und wobei das Fenster eine periodische räumlich-variierende Transmission aufweist.

11. Verfahren nach Anspruch 9 oder 10, ferner umfassend ein Empfangen von Antwortdaten von dem Detektor, wobei die Daten auf das zeitlich-variierende optische Signal und das zweite optische Signal hinweisen, und ein Bestimmen des Vorhandenseins der Probenverbindung ein Verarbeiten der Antwortdaten umfasst.

12. Verfahren nach einem der Ansprüche 9 bis 11, wobei das Verarbeiten der Antwortdaten ein automatisches Korrelieren der Antwortdaten umfasst, und ein Bestimmen des Vorhandenseins der Probenverbindung basierend zumindest teilweise auf einem Teil der automatisch korrelierten Daten, die auf das erste Signal hindeuten.

13. Verfahren, umfassend: Kombinieren einer Probenverbindung, einer magnetempfindlichen Kennzeichnung, umfassend ein Bindungsreagens, das imstande ist die Probenverbindung zu binden, und einer zweiten Kennzeichnung, umfassend ein Bindungsreagens, das imstande ist die Probenverbindung zu binden, Aussetzen der Mischung an ein Magnetfeld, Beleuchten der Mischung mit Licht, um ein erstes optisches Signal von der zweiten Kennzeichnung zu erzeugen, die mit der Probenverbindung verbunden ist, und ein zweites optisches Signal von der zweiten Kennzeichnung, die nicht mit der Probenverbindung verbunden ist, Modulieren einer Zeitfrequenz des ersten optischen Signals, und Bestimmen des Vorhandenseins der Probenverbindung basierend zumindest teilweise auf dem ersten optischen Signal.

14. Verfahren nach Anspruch 13, wobei das Modulieren der Zeitfrequenz ein Fortpflanzen der ersten und zweiten optischen Signale entlang eines Lichtwegs mit einer räumlich-variierenden Transmission entlang einer Achse senkrecht zu dem Lichtweg umfasst, wobei der Lichtweg ein Fenster zu der Erfassungszone umfasst, das Fenster eine räumlich-variierende Transmission aufweist.

**Revendications**

1. Dispositif pour analyser un composé échantillon, comprenant : une zone de détection configurée pour recevoir une matière échantillon comprenant : un marqueur magnétiquement sensible comprenant un réactif de liaison apte à se lier au composé échantillon, et un second marqueur comprenant un réactif de liaison apte à se lier au composé échantillon, un générateur de champ magnétique configuré pour soumettre la matière échantillon dans la zone de détection à un champ magnétique, une source de lumière configurée pour éclairer la matière échantillon au sein de la zone de détection, un système de détection comprenant un détecteur de lumière ; dans lequel : le dispositif définit un trajet optique entre la zone de détection et le détecteur, le trajet optique ayant une transmittance variant dans l'espace le long d'un axe normal par rapport au trajet optique, le détecteur est configuré pour recevoir un signal optique variant dans le temps comprenant un premier signal optique provenant du second marqueur associé au composé échantillon et un second signal optique provenant du second marqueur non associé au composé échantillon, et le système de détection est configuré pour déterminer la présence du composé échantillon sur la base au moins en partie du signal optique variant dans le temps.

2. Dispositif selon la revendication 1, dans lequel la transmittance optique variant dans l'espace du trajet optique est périodique.

3. Dispositif selon la revendication 1 ou 2, dans lequel le champ magnétique est un champ magnétique alternatif.

4. Dispositif selon la revendication 1, dans lequel le générateur de champ magnétique est configuré pour soumettre la matière échantillon dans la zone de détection à un champ magnétique variant dans le temps.

5. Dispositif selon la revendication 1, 2, 3 ou 4, dans lequel le détecteur est configuré pour délivrer en sortie des données de réponse variant dans le temps indicatives du signal optique variant dans le temps et le système de détection comprend en outre un processeur configuré pour traiter les données de réponse variant dans le temps afin de déterminer la présence du composé échantillon et dans lequel le processeur est configuré pour autocorréler les données de réponse variant dans le temps et pour déterminer la présence du composé échantillon sur la base au moins en partie d'une partie des données autocorrélées qui sont indicatives du premier signal.

6. Dispositif selon la revendication 5, dans lequel le système de détection comprend un filtre électronique configuré pour recevoir les données de réponse variant dans le temps, le filtre électronique ayant une réponse en fréquence configurée pour laisser passer des fréquences correspondant au premier signal et pour rejeter de manière relative des fréquences correspondant au second signal.

7. Dispositif selon l'une quelconque des revendications 1 à 6, dans lequel le trajet optique comprend une fenêtre ayant une transmittance optique variant dans l'espace le long d'un axe normal par rapport au trajet optique et le détecteur de lumière est configuré pour détecter la lumière qui est passée au travers de la fenêtre à partir de la zone de détection, ou dans lequel la fenêtre comprend une transmittance optique spatiale périodique.

8. Dispositif selon l'une quelconque des revendications 1 à 7, dans lequel le second marqueur est fluorescent lors de l'éclairement avec une longueur d'onde émise par la source de lumière et le détecteur de lumière est configuré pour détecter la fluorescence et dans lequel le réactif de liaison du second marqueur est apte à se lier spécifiquement au BNP, au proBNP ou au NT-proBNP.

9. Procédé comprenant les étapes consistant à : combiner un composé échantillon, un marqueur magnétiquement sensible comprenant un réactif de liaison apte à se lier au composé échantillon, et un second marqueur comprenant un réactif de liaison apte à se lier au composé échantillon; soumettre le mélange à un champ magnétique variant dans le temps, éclairer le mélange avec de la lumière, recevoir un signal optique variant dans le temps comprenant un premier signal optique provenant du second marqueur lié au composé échantillon et un second signal provenant du second marqueur non lié au composé échantillon, et déterminer la présence du composé échantillon sur la base au moins en partie du signal optique variant dans le temps, le procédé comprenant en outre la migration magnétique d'au moins une partie de la matière échantillon vers la zone de détection avant la réception du signal optique variant dans le temps et du second signal optique.

10. Procédé selon la revendication 9, dans lequel la détection comprend la détection du signal optique variant dans le temps après que le signal est passé le long d'un trajet optique ayant une transmittance optique variant dans l'espace le long d'un axe normal par rapport au trajet optique, ou dans lequel la détection comprend la détection du signal optique variant dans le temps après que le signal est passé le long du trajet optique à travers une fenêtre ayant une transmittance variant dans l'espace, et dans lequel la fenêtre a une transmittance périodique variant dans l'espace.

11. Procédé selon la revendication 9 ou 10, comprenant en outre la réception de données de réponse en provenance du détecteur, des données indicatives du signal optique variant dans le temps et du second signal optique, et la détermination de la présence du composé échantillon comprend le traitement des données de réponse.

12. Procédé selon l'une quelconque des revendications 9 à 11, dans lequel le traitement des données de réponse, comprend l'autocorrélation des données de réponse et la détermination de la présence du composé échantillon sur la base au moins en partie d'une partie des données autocorrélées qui sont indicatives du premier signal.

13. Procédé comprenant les étapes consistant à : combiner un composé échantillon, un marqueur magnétiquement sensible comprenant un réactif de liaison apte à se lier au composé échantillon, et un second marqueur comprenant un réactif de liaison apte à se lier au composé échantillon, soumettre le mélange à un champ magnétique, éclairer le mélange avec une lumière pour produire un premier signal optique à partir du second marqueur lié au composé échantillon et un second signal optique à partir du second marqueur non lié au composé échantillon, moduler une fréquence temporelle du premier signal optique, et déterminer la présence du composé échantillon sur la base au moins en partie du premier signal optique.

14. Procédé selon la revendication 13, dans lequel la modulation de la fréquence temporelle comprend la propagation des premier et second signaux optiques le long d'un trajet optique ayant une transmittance variant dans l'espace le long d'un axe normal par rapport au trajet optique, dans lequel le trajet optique comprend une fenêtre sur la zone de détection, la fenêtre ayant une transmittance variant dans l'espace.

**FIG. 1**

**FIG. 2**

**FIG. 3A**

**FIG. 3B**

**FIG. 4A**

**FIG. 4B**

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040058458 A
- US 20050147963 A
- US 20050100930 A
- US 5348876 A
- US 6682648 B
- US 6406913 B
- US 20050149169 A
- US 20050148096 A
- US 20050142549 A
- US 20050074748 A
- US 20050106652 A
- US 6143514 A

**Non-patent literature cited in the description**

- **M. Kinjo ; R. Rigler.** *Nucl. Acids Res.,* 1995, vol. 23, 1795-1799
- **R. Rigler ; E. L. Elson.** Fluorescence Correlation Spectroscopy: Theory and Applications. Springer, 2001
- **Oleg Krichevsky et al.** *Rep. Prog. Phys.,* 2002, vol. 65, 251-297
- **Schwille, P. et al.** *Biophys. J.,* 1997, vol. 72, 1878-1886
- *Proc. Natl. Acad. Sci.,* 1994, vol. 91, 5426-5430
- **Khanna, P.L. ; Coty, W.A.** Methods of Immunological Analysis. VCH Verlagsgesellschaft MbH, 1993, vol. 1, 416-426
- **Coty, W.A. ; Loor, R. ; Powell, M. ; Khanna, P.L.** *J. Clin. Immunoassay,* 1994, vol. 17 (3), 144-150
- **Coty, W.A. ; Shindelman, J. ; Rouhani, R. ; Powell, M.J.** *Genetic Engineering News,* 1999, vol. 19 (7